(19) **Europäisches Patentamt**
**European Patent Office**
**Office européen des brevets**

(11) **EP 1 684 654 B1**

(12) **EUROPEAN PATENT SPECIFICATION**

(45) Date of publication and mention
of the grant of the patent:
**12.11.2014 Bulletin 2014/46**

(51) Int Cl.:
**A61B 18/12** (2006.01)

(21) Application number: **03789916.8**

(86) International application number:
**PCT/US2003/037310**

(22) Date of filing: **21.11.2003**

(87) International publication number:
**WO 2005/046496 (26.05.2005 Gazette 2005/21)**

(54) **AUTOMATIC CONTROL SYSTEM FOR AN ELECTROSURGICAL GENERATOR**

AUTOMATISCHES STEUERSYSTEM FÜR EINEN ELEKTROCHIRURGISCHEN GENERATOR

SYSTEME DE COMMANDE AUTOMATIQUE POUR GENERATEUR ELECTROCHIRURGICAL

(84) Designated Contracting States:
**DE ES FR GB IE IT**

(30) Priority: **30.10.2003 US 515816 P**

(43) Date of publication of application:
**02.08.2006 Bulletin 2006/31**

(73) Proprietor: **Covidien AG**
**8212 Neuhausen am Rheinfall (CH)**

(72) Inventors:
• **WHAM, Robert Hartzell**
**Boulder, CO 80305 (US)**
• **STURM, Thomas, A.**
**Erie, CO 80516 (US)**

(74) Representative: **Hoffmann Eitle**
**Patent- und Rechtsanwälte PartmbB**
**Arabellastraße 30**
**81925 München (DE)**

(56) References cited:
**US-A- 5 931 836**

• **CHICHARO J F ET AL: "A sliding Goertzel algorithm" SIGNAL PROCESSING, ELSEVIER SCIENCE PUBLISHERS B.V. AMSTERDAM, NL, vol. 52, no. 3, 1 August 1996 (1996-08-01), pages 283-297, XP004012139 ISSN: 0165-1684**
• **OGDEN F: "GOERTZEL ALTERNATIVE TO THE FOURIER TRANSFORM" ELECTRONICS WORLD (INCL. WIRELESS WORLD), REED BUSINESS PUBLISHING, SUTTON, SURREY, GB, vol. 99, no. 1687, 1 June 1993 (1993-06-01), pages 485-487, XP000303838 ISSN: 0959-8332**

Printed by Jouve, 75001 PARIS (FR)

**Description**

**BACKGROUND OF THE INVENTION**

1. Technical Field

The present disclosure relates to electrosurgery. More particularly, the present disclosure relates to an automatic control system for an electrosurgical generator.

2. Background of Related Art

Surgeons have tried to deal with energy application by adjusting the basic power level of the electrosurgical generator and using a hand or foot switch to control the power applied over time. Unfortunately, that technique often leads to unintended power delivery or undesired duration of power delivery to the surgical site. Surgeons also experience difficulty in repeatedly and/or consistently desiccating tissue to the desired levels due to the user's reaction time and/or machine response time when manual or foot activated switches are used for manual control. In addition, during endoscopic procedures, visual and tactile feedback is diminished.

A circuit for automatically controlling the output of an electrosurgical generator is disclosed in U. S. Patent No. 6,210, 403 to Klicek, currently owned, and assigned to Sherwood Services AG.

U.S. Patent No. 6,210, 403 relates to an electrosurgical generator control, which is responsive to the tissue impedance between the active and return electrodes during desiccation.

A method for tone detection using the Goertzel algorithm is disclosed in an article entitled *The Goertzel Algorithm* by Kevin Banks (*The Goertzel Algorithm* by Kevin Banks, <http://www.embedded.com/showArticle.jhtml?articleID=9900772>, last visited on July 24, 2003). The Banks' article relates to using a modified Goertzel algorithm for determining whether a tone of a specific frequency is present. The Goertzel algorithm calculates both the magnitude and the phase of signal at a specific frequency and is functionally equivalent to performing a Discrete Fourier Transform (DFT) at a single frequency, but is much less computationally demanding. The DFT is a method for calculating the magnitude and phase of a band of frequencies of interest. An N-point DFT is computationally demanding, but will calculate the real and imaginary frequency terms for all the frequencies up to half the sampling rate of the signal.

According to Banks, using a modified Goertzel algorithm is preferable in applications requiring tone detection such as DTMF, call progress decoding, and frequency response measurements. However, the modified Goertzel algorithm proposed by Banks does not provide the real and imaginary frequency components of the sampled waveform. As a result, the modified Goertzel algorithm is unsuited for determining the phase of the waveform.

It is an object of the present disclosure to provide an automatic control system that uses fewer computational steps.

Another object of the present disclosure is to provide an automatic control system that measures the power delivered to a patient.

Yet a further object of the present disclosure is to provide an automatic control system that is adaptable to both monopolar and bipolar electrosurgical generator configurations.

It is a further object of the present disclosure to provide an automatic control system that adjusts the power delivered to a patient by an electrosurgical generator.

Document US - A - 5 931 836 discloses the most relevant prior art.

**SUMMARY**

The invention is defined in claim 1.

The present disclosure relates to an automatic control system for an electrosurgical generator which includes voltage and current sensing circuits, a processing circuit, an output determining circuit, and a control circuit. The voltage and current sensing circuits produce voltage and current signals that are representative of the voltage and current present in the output of the electrosurgical generator. These signals are coupled to the processing circuit that uses a Goertzel algorithm to determine the phase difference between the voltage waveform and the current waveform according to circuitry within the processing circuit.

The processing circuit produces a phase difference signal that is communicated to the output determining circuit for determining the output of the electrosurgical generator. The output determining circuit produces an output signal that is compared to a reference signal in the control circuit. The control circuit determines the difference between the output signal and the reference signal and generates a feedback signal that is representative of the difference. The feedback signal is communicated to a drive control circuit for controlling the output of a drive circuit.

The Goertzel algorithm determines phase angle between the voltage waveform and the current waveform.

Advantageously, the phase angle is used to compensate for energy delivery at the operating site. It is also contemplated that the phase angle can be utilized to provide feedback to the generator about tissue relating to at least one of: tissue change over time, tissue impedance, tissue type, tissue cycle completion.

**BRIEF DESCRIPTION OF THE DRAWINGS**

Embodiments of the presently disclosed are described herein with reference to the drawing, wherein:

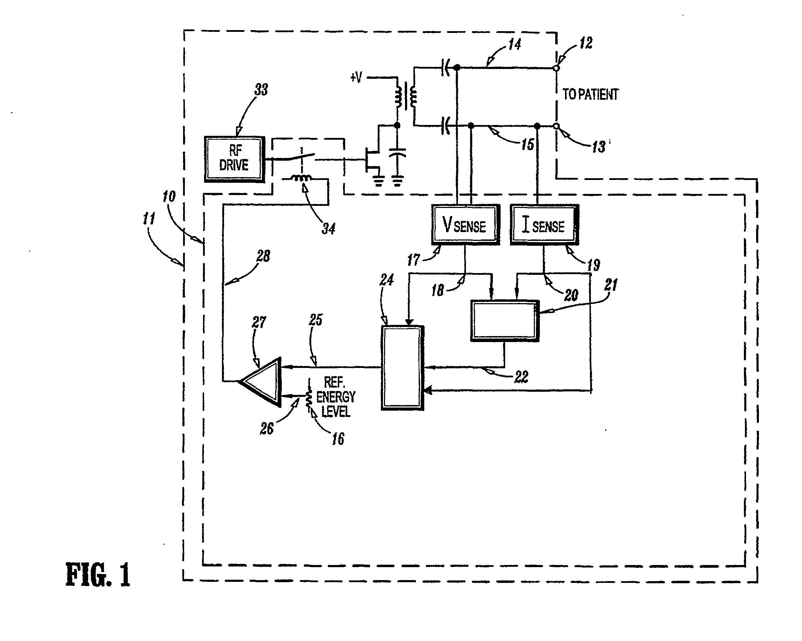

FIG. 1 is block diagram of an automatic control system for an electrosurgical generator in accordance with an embodiment of the present disclosure.

**DETAILED DESCRIPTION**

Embodiments of the presently disclosed automatic control system will now be described in detail with reference to the drawing where, in FIG. 1, an exemplary embodiment of the presently disclosed automatic control system 10 is illustrated. Automatic control system 10 is ideally disposed within an electrosurgical generator 11. Electrosurgical generator 11 includes a user control 16 preferably on its front panel accessible to the doctor for setting the output level desired for a particular electrosurgical procedure. User control 16 may be a knob, a slider, or other structures and/or devices as is known in the art for use by the doctor to set a reference signal 26 indicative of the desired output.

A voltage sensing circuit 17 has an isolation transformer, which acts as an inductive pickup. Its primary side is electrically connected between leads 14 and 15 for inducing a voltage signal 18 on the secondary windings thereby responding to the high frequency electrosurgical energy supplied by electrosurgical generator 11 flowing through leads 14 and 15. A current sensing circuit 19 responds to high frequency electrosurgical energy supplied by electrosurgical generator 11 and flowing through return lead 15. Current sensing circuit 19 provides a current signal 20 as an instantaneous output representative of the current passing therethrough. Preferably, voltage signal 18 and current signal 20 are AC waveforms that are representative of the output of leads 14 and 15.

Operatively connected to leads 14 and 15 are electrodes 12 and 13. Electrodes 12 and 13 are used to provide the output of electrosurgical generator 11 to a patient. In a bipolar configuration, electrodes 12 and 13 are both present in an electrosurgical instrument (not shown), which is used at a surgical site of the patient with electrode 13 providing the return path for the output of electrosurgical generator 11.

In a monopolar configuration, the electrosurgical instrument (not shown) includes one electrode 12 while electrode 13 is connected to a surface near the patient and provides the return path. The active ends of electrodes 12 and 13 are electrically connected to electrosurgical generator 11 by one or more conductive cables. Although monopolar and bipolar configurations are used in electrosurgical generators, they are electrically equivalent and equally suited for use with automatic control system 10 of the present disclosure.

Voltage sensing circuit 17 and current sensing circuit 19 are operatively coupled to a processing circuit 21. In a preferred embodiment, processing circuit 21 includes one or more digital signal processors (DSP) and associated circuitry. The DSPs may be upgradeable using flash ROM as is known in the art. Upgrades for the DSPs may be stored on computer readable media such as magnetic disks, optical disks, magnetic tape, or other media as is known in the art. Processing circuit 21 simultaneously receives voltage signal 18 and current signal 20.

Processing circuit uses the Goertzel algorithm for processing voltage and current signals 18, 20. The Goertzel algorithm is advantageously implemented as a second order recursive infinite impulse response filter, as shown below.

The Goertzel algorithm is defined by the equation :

$$H_{f_i}(z) = \frac{1 - e^{\frac{2\pi f_i}{f_s}} z^{-1}}{1 - 2\cos\left(\frac{2\pi f_i}{f_s}\right) z^{-1} + z^{-2}}$$

Where $f_i$ is the frequency of interest and $f_R$ is the sampling frequency. Second Order Recursive Goertzel Filter

## Second Order Recursive Goertzel Filter

Input                                                                                    Output

The Goertzel algorithm is implemented digitally as:

$$v_k[n] = x[n] + 2\cos\left(\frac{2\pi k}{N}\right)v_k[n-1] - v_k[n-2]$$

Since the output frequency of electrosurgical generator 11 is known, and preferably, about 470 KHz, the digitally implemented Goertzel algorithm calculates the real and imaginary frequency components of the known waveform using the following formulae:

$$\text{Real} = (v_k[n-1] - (v_k[n-2]*\cos(2\pi k/N))$$

$$\text{Imaginary} = (v_k[n-2]*\sin(2\pi k/N))$$

$$\text{Magnitude} = \text{square\_root}(\text{Real}^2 + \text{Imaginary}^2)$$

$$\text{Phase} = \text{ATAN}(\text{Imaginary/Real})$$

The DSPs of processing circuit 21 calculates the Voltage_Phase for voltage signal 18 and the Current_Phase for current signal 20 according to the above-mentioned formulae. Additionally, the phase shift, preferably in radians, between voltage signal 18 and current signal 20 can then be calculated by applying the algorithm on voltage signal 18 and current signal 20 concurrently and subtracting the difference in the phases as follows:

$$\text{Phase\_Difference} = \text{Current\_Phase} - \text{Voltage\_Phase}.$$

This phase calculation is implemented to calculate the phase differential between voltage signal 18 and current signal 20. In the preferred embodiment, the DSPs of processing circuit 21 include the Goertzel algorithm along with associated processing software to determine the phase difference between voltage signal 18 and current signal 20. Additionally, processing circuit 21 determines a magnitude value of both voltage and current signals 18, 20 and communicates these values along with the Phase_Difference to an output determining circuit 24 as phase difference signal 22.
In one embodiment, output determining circuit 24 includes a microprocessor with associated circuitry for calculating the dosage (current, power or voltage) output of electrosurgical generator 11 using the calculated Phase_Difference and values of the voltage and current outputs of electrosurgical generator 11. In an AC circuit, power is determined by the formula P=EI cos (q), where P is the power measured in watts, E is a voltage value, I is a current value, and q is the Phase_Difference between the voltage and current waveforms.

By advantageously using the Goertzel algorithm for a single known value of frequency, automatic control system 10 of the present disclosure calculates the output for electrosurgical generator 11 using fewer computational steps than a DFT. More particularly, due to the frequency of the output and the selected sampling rate for the voltage and current components of the output, there is insufficient bandwidth to use a DFT to determine the Phase_Difference. However, processing circuit 21, according to the present disclosure, determines the Phase_Difference using the Goertzel algorithm, thereby using fewer computational steps and within the existing bandwidth. As used herein, bandwidth refers to the time between the voltage and/or current samples acquired by voltage and current sensing circuits 17, 19.

Preferably, automatic control system 10 additionally calculates the output of electrosurgical generator 11 and performs any necessary adjustments to the output within the existing bandwidth. In the preferred embodiment, after automatic control system 10 calculates the output and performs any necessary adjustments, there is additional bandwidth available before the next sample of the output is taken. Furthermore, by using fewer computational steps to determine Phase_Difference, a minimum amount of data is lost between samples.

The Goertzel algorithm is used to determine the phase angle or Phase_Difference between the voltage waveform and the current waveform. Advantageously, the Phase_Difference is used to compensate for energy delivery at the operating site. It is also contemplated that the Phase_Difference can be utilized to provide feedback to the generator 11 about tissue relating to at least one of: tissue change over time, tissue impedance, tissue type, tissue cycle completion.

Extra bandwidth between samples of the output is advantageously utilized to perform additional calculations, perform additional control functions, or allow the output frequency of electrosurgical generator 11 to be increased. By way of example, such additional calculations include average values of voltage and current, peak values of voltage and current, and root mean square values of voltage and current. It is contemplated that, additional control functions may include calibration of system components and adjusting system parameters for cable compensation.

Output determining circuit 24 includes circuitry for determining electrosurgical generator's 11 output. Preferably, output determining circuit 24 includes a processor and associated circuitry for determining the current, voltage, and/or power delivered to the patient. An output signal 25 is generated by output determining circuit 24 and is coupled to an input of a control circuit 27. In a preferred embodiment, voltage and current signals 18, 20 are also communicated to output determining circuit 24. The circuitry in output determining circuit 24 determines the output of electrosurgical generator 11 using voltage and current signals 18, 20 in conjunction with phase difference signal 22. The output of electrosurgical generator 11 is represented by a value of output signal 25.

Control circuit 27 has at least two inputs where a first input is output signal 25 and a second input is a reference signal 26. Reference signal 26 is controlled by the setting of user control 16 and it establishes a reference value for control circuit 27. In a preferred embodiment, control circuit 27 includes at least one DSP and associated circuitry for determining the difference between output signal 25 and reference signal 26. A feedback signal 28 is generated by control circuit 27 where the feedback signal 28 is representative of the difference between output signal 25 and reference signal 26.

Feedback signal 28 is operatively coupled to a drive control circuit 34 for controlling the output of a drive circuit 33. Drive control circuit 34 includes structure and/or circuitry for controlling the output of drive circuit 33. In one embodiment, drive control circuit 34 controls an input to drive circuit 33 for adjusting the output of drive circuit 33 according to a value of feedback signal 28. Alternatively, drive control circuit 34 controls the output of drive circuit 33 by adjusting the biasing of associated circuitry in drive circuit 33 according to a value of feedback signal 28, thereby controlling its output.

During operation of electrosurgical generator 11, drive circuit 33 produces an output, or drive signal, that is coupled to a first winding of a transformer. A portion of the output present on the first winding of the transformer is coupled to a second winding of the transformer that is electrically communicated to leads 14 and 15. Leads 14 and 15 are electrically connected to electrodes 12 and 13 for operating an electrosurgical instrument (not shown) during an electrosurgical procedure. The output present on leads 14 and 15 is sampled by voltage sensing circuit 17 and current sensing circuit 19. As discussed in detail above, the Phase_Difference between the output voltage waveform and output current waveform is determined by processing circuit 21 and the output of electrosurgical generator 11 is determined by output determining circuit 24.

As output from electrosurgical generator 11 increases, the values of voltage signal 18 and current signal 20 also increase in a proportional relationship. Output determining circuit 24 receives phase difference signal 22 from processing circuit 21 and determines the change in the output. Accordingly, an increase in output is reflected in an increase in output signal 25 that is coupled to control circuit 27. Due to the increase in output signal 25, the difference between output signal 25 and reference signal 26 decreases resulting in a decreased feedback signal 28.

When output signal 25 is substantially equal to reference signal 26, feedback signal 28 is essentially zero. Additionally, the substantial equality of these signals indicates that electrosurgical generator 11 is producing the desired output for the selected electrosurgical procedure.

Other uses for electrosurgical generator 11 including automatic control system 10 are envisioned to be within the scope of this disclosure. Such applications include procedures where fine control and accuracy of delivered output is desirable. These applications include neurosurgical applications, ligasure sealing, thoracic and throat procedures, ocular surgery, procedures on small structures, and neonatal procedures. The determination of the Phase_Difference will allow output compensation so that with a known cable and handset, the output delivered to the patient can be more accurately calculated.

Further still, since automatic control system 10 determines the Phase Difference between the voltage and current components of the output, this information may be coupled with known values of a handset and cable electrical characteristics (i. e. resistance, capacitance, and inductance) to determine the distance between the electrosurgical instrument and the surface of the patient. This is especially advantageous in a coagulation procedure where an electrosurgical generator is used in conjunction with an electrosurgical pencil (i. e.monopolar mode of operation) disposed above the surface of the patient. In this procedure, the electrosurgical generator typically produces a high voltage that arcs from the electrosurgical pencil to the surface of the patient, thereby coagulating affected tissue. By determining the distance between the electrosurgical pencil and the patient, automatic control system 10 can adjust the power output to a desired value that is sufficient to coagulate the affected tissue without producing additional power.

A Goertzel algorithm is described herein as one low computation algorithm for determining the magnitude and phase components of a narrow band sinusoidal signal. Use of the Goertzel algorithm is a feature of claim 1.

Although the illustrative embodiments of the present disclosure have been described herein with reference to the accompanying drawings, it is to be understood that the disclosure is not limited to those precise embodiments, and that various other changes and modifications may be affected therein by one skilled in the art.

## Claims

1. A system for controlling an output of an electrosurgical generator (11) comprising:

a drive circuit (33) for generating an output, the output being responsive to a feedback signal and operatively coupled to at least one electrode of the electrosurgical generator;
at least one sensing circuit (17,19) operatively coupled to the at least one electrode for generating a first signal, corresponding to a value of a voltage waveform present on the at least one electrode and a second signal corresponding to a value of a current waveform present on the at least one electrode;
a processing circuit (21) for receiving the first and second signals, the processing circuit including associated circuitry configured to use the Goertzel algorithm to determine a phase difference between the voltage waveform and the current waveform;
a determining circuit (24) in communication with the processing circuit, the processing circuit configured to communicated the phase difference to the determining circuit, and the determining circuit is configured to use the phase difference to generate an output signal; and
a control circuit (27) for generating the feedback signal, the feedback signal representative of a difference between a value of the output signal and a reference value, the feedback signal operatively coupled to the drive circuit.

2. A system according to claim 1, wherein the processing circuit includes at least one digital signal processor.

3. A system according to any preceding claim, wherein the phase difference is used to compensate for energy delivery at the operating site.

4. A system according to any preceding claim, wherein the phase difference provides feedback to the generator about tissue relating to at least one of: tissue change over time, tissue impedance, tissue type, tissue cycle completion.

5. A system according to any preceding claim, wherein the at least one sensing circuit includes a voltage sensing circuit and/or a current sensing circuit.

## Patentansprüche

1. System zum Steuern einer Ausgabe eines elektrochirurgischen Generators (11), mit:

einem Treiberschaltkreis (33) zum Erzeugen einer Ausgabe, wobei die Ausgabe von einem Feedbacksignal abhängig ist und betriebsfähig mit mindestens einer Elektrode des elektrochirurgischen Generators gekoppelt ist;

mindestens einem Messschaltkreis (17, 19), der betriebsfähig mit der mindestens einen Elektrode zum Erzeugen eines ersten Signals gekoppelt ist, das mit einem Wert einer Spannungswellenform korrespondiert, die an der mindestens einen Elektrode vorliegt, und eines zweiten Signals, das mit einem Wert einer Stromwellenform korrespondiert, die an der mindestens einen Elektrode vorliegt;

einem Verarbeitungsschaltkreis (21) zum Empfangen des ersten und zweiten Signals, wobei der Verarbeitungsschaltkreis zugehörige Schaltungen aufweist, die eingerichtet sind, den Goertzelalgorithmus zu verwenden, um einen Phasenunterschied zwischen der Spannungswellenform und der Stromwellenform zu bestimmen;

einem Bestimmungsschaltkreis (24), der mit dem Verarbeitungsschaltkreis in Verbindung steht, wobei der Verarbeitungsschaltkreis eingerichtet ist, dem Bestimmungsschaltkreis den Phasenunterschied zu übermitteln, und der Bestimmungsschaltkreis eingerichtet ist, den Phasenunterschied zu verwenden, um ein Ausgangssignal zu erzeugen; und

einem Steuerschaltkreis (27) zum Erzeugen des Feedbacksignals, wobei das Feedbacksignal einen Unterschied zwischen einem Wert des Ausgangssignals und einem Referenzwert wiedergibt, wobei das Feedbacksignal betriebsfähig mit dem Treiberschaltkreis gekoppelt ist.

2. System nach Anspruch 1, bei dem der Verarbeitungsschaltkreis mindestens einen digitalen Signalprozessor aufweist.

3. System nach einem der vorstehenden Ansprüche, bei dem ein Phasenunterschied verwendet wird, um eine Energiezufuhr bei einem Eingriffsort zu kompensieren.

4. System nach einem der vorstehenden Ansprüche, bei dem der Phasenunterschied dem Generator ein Feedback über Gewebe bereitstellt, das mit einer Gewebeveränderung über die Zeit, einer Gewebeimpedanz, einer Gewebeart und/oder einem Gewebezyklusabschluss zusammenhängt.

5. System nach einem der vorstehenden Ansprüche, bei dem der mindestens eine Messschaltkreis einen Spannungsmessschaltkreis und/oder einen Strommessschaltkreis aufweist.


**Revendications**

1. Système de commande d'une sortie d'un générateur électro-chirurgical (11) comprenant :

un circuit d'entraînement (33) pour la génération d'une sortie, la sortie réagissant à un signal de rétroaction et étant fonctionnellement couplée à au moins une électrode du générateur électro-chirurgical ;

au moins un circuit de détection (17, 19) fonctionnellement couplé à au moins une électrode précitée pour produire un premier signal, correspondant à une valeur d'une forme d'onde de tension présente sur la au moins une électrode, et un deuxième signal correspondant à une valeur d'une forme d'onde de courant présente sur la au moins une électrode ;

un circuit de traitement (21) destiné à recevoir les premier et deuxième signaux, le circuit de traitement incluant des circuits associés configurés pour utiliser l'algorithme de Goertzel afin de détermine une différence de phase entre la forme d'onde de tension et la forme d'onde de courant ;

un circuit de détermination (24) en communication avec le circuit de traitement, le circuit de traitement étant configuré pour communiquer la différence de phase au circuit de détermination, et le circuit de détermination est configuré pour utiliser la différence de phase pour produire un signal de sortie ; et

un circuit de commande (27) pour produire le signal de rétroaction, le signal de rétroaction étant représentatif d'une différence entre une valeur du signal de sortie et une valeur de référence, le signal de rétroaction étant fonctionnellement couplé au circuit d'entraînement.

2. Système selon la revendication 1, dans lequel le circuit de traitement comprend au moins un processeur de signaux numériques.

3. Système selon l'une quelconque des revendications précédentes, dans lequel la différence de phase est utilisée pour compenser la délivrance d'énergie au site d'opération.

4. Système selon l'une quelconque des revendications précédentes, dans lequel la différence de phase fournit une réaction au générateur autour du tissu se rapportant à au moins un de : changement de tissu avec le temps, impédance du tissu , type de tissu, achèvement de cycle de tissu.

**5.** Système selon l'une quelconque des revendications précédentes, dans lequel le au moins un circuit de détection inclut un circuit de détection de tension et/ou un circuit de détection de courant.

**FIG. 1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6210403 A
- US 5931836 A